# EUROPÄISCHE PATENTANMELDUNG

(11) **EP 3 811 820 A1**
(43) Veröffentlichungstag der Anmeldung: **28.04.2021**
(21) Anmeldenummer: 20020391.7
(22) Anmeldetag: 11.06.2014
(51) Int. Cl.: A46B 15/00, A61C 17/22

(54) **VERFAHREN ZUR BESTIMMUNG VON BEWEGUNGSMUSTERN BEI EINER ZAHNBEHANDLUNG**

(30) Priorität: 19.06.2013 DE 102013010292; 18.09.2013 DE 102013015537; 17.12.2013 DE 102013021492
(62) Teilanmeldung aus: 14733110.2
(71) Anmelder: Valutis GmbH, 76863 Herxheim (DE)
(72) Erfinder: Ohmer, Benjamin, 81927 München (DE)
(74) Vertreter: Kehl, Ascherl, Liebhoff & Ettmayr Patentanwälte Partnerschaft mbB

(57) **Zusammenfassung**

Die Erfindung bezieht sich auf ein Verfahren zum zumindest teilweisen Bestimmen eines sich bei einer Zahnbehandlung, insbesondere Zahnreinigung, ergebenden Bewegungsmusters eines Zahnbehandlungsmittels, insbesondere einer Zahnbürste, und umfasst mindestens die Schritte: Bewegen des Zahnbehandlungsmittel zum Behandeln von Oberflächenanteilen der Zähne zumindest in einer X-/Y-Ebene, wobei mittels einer optischen Detektionseinrichtung Daten bezüglich mindesten eines Bewegungsparameters, insbesondere die Bewegungsrichtung oder - rotation, die Beschleunigung, der Weg und/oder die Geschwindigkeit, des Zahnbehandlungsmittels gegenüber einem sich mit dem Kopf der behandelten Person mitbewegenden Bezugssystems, erfasst werden, und Bereitstellen der erfassten Daten an eine Prozessoreinrichtung zur Bestimmung des Bewegungsmusters.

## Beschreibung

Die Erfindung kann sich auf ein System und ein Verfahren zur Bestimmung von Bewegungsmustern bei einer Zahnbehandlung, insbesondere der Zahnreinigung, beziehen. Aufgrund der relativ langen Intervalle von bis zu mehreren Monaten oder sogar Jahren zwischen den einzelnen Zahnarztbesuchen ergibt sich das Problem, dass die Anweisungen des Zahnarztes bezüglich Häufigkeit, Dauer und Art der Behandlung leicht vergessen werden oder sich mit der Zeit Bewegungsabläufe einspielen, die nicht optimal sind, wodurch die Mundhygiene nicht optimal gewährleistet wird oder Schädigungen des Mundraums, insbesondere fester und/oder weicher Mundbestandteile, wie z.B. der Zähne, des Kiefers und/oder des Zahnfleisches, bewirkt werden.

Die Druckschrift EP 1 379 149 B1 offenbart ein Verfahren zur Überwachung der Position einer Zahnbürste in Bezug zu Zähnen einer Person, wobei das Verfahren umfasst: Bereitstellen einer Zahnbürste mit einem ersten Positionssensor, wobei der erste Positionssensor zumindest sensibel auf Änderungen der Position und Orientierung reagiert, Bereitstellen eines zweiten Positionssensors in fester Positionsbeziehung zu den Zähnen, wobei der zweite Positionssensor sensibel auf Änderungen der Position und Orientierung ; Übertragen der Ausgabe des ersten Positionssensors und des zweiten Positionssensors zur Verarbeitungsvorrichtung, und die Verarbeitungsvorrichtung vergleicht die beiden Sensorausgänge, um die Position der Zahnbürste in Bezug zu den Zähnen über einen Zeitraum zu überwachen.

Dieses System ist nachteilig, da aufwendige Sensoren definiert an einem Gesicht angebracht werden müssen, wodurch das System teuer und komplex ist, wodurch eine hohe Marktakzeptanz erschwert ist.

Yu-Chen Chang et. al. beschreiben in dem Artikel"Playful Toothbrush: UbiComp Technology for Teaching Tooth Brushing to Kindergarten Children" ein System, bei dem die Bewegung der Zahnbürste optisch erfasst wird. Dieses System ist nachteilig, da die erfassten Bewegungen Undefiniert im Raum erfolgen, wodurch eine Bewegung des Kopfes eine Verfälschung der erfassten Daten bewirkt. Ferner werden aufwendige Modifikationen der Zahnbürste vorausgesetzt, wodurch das System komplex und teuer ist und somit ebenfalls für einen Massenmarkt unzugänglich erscheint. Zudem muss das System raumfest installiert werden, damit eine Kamera definiert die modifizierte Zahnbürste erkennen kann.

Weiterhin basieren beide Systeme auf vorgegebenen Datensätzen, wodurch eine Verbesserung des Wissens über Zahnputzbewegungen nicht möglich ist. Es ist somit Aufgabe der vorliegenden Erfindung ein Verfahren und ein System bereitzustellen, das eine Optimierung der Mundgesundheit, insbesondere der Mundhygiene, der Zahngesundheit, der Kiefergesundheit und/oder der Zahnfleischgesundheit ermöglicht. Bevorzugt soll die Erfindung auch eine Kontrolle, insbesondere zur kontinuierlichen oder permanenten Kontrolle, bzw. eine Überwachung, insbesondere zur kontinuierlichen oder permanenten Überwachung, der Mundhygiene ermöglichen. Weiter bevorzugt soll die Erfindung möglichst einfach, günstig und von möglichst vielen Personen einsetzbar sein.

Das erfindungsgemäße Verfahren umfasst bevorzugt den Schritt des Bewegens des Zahnbehandlungsmittel zum Behandeln von Oberflächenanteilen der Zähne zumindest in einer X-/Y- Ebene, wobei mittels mindestens einer optischen Detektionseinrichtung Daten bezüglich mindesten eines Bewegungsparameters, insbesondere die Bewegungsrichtung oder -rotation, die Beschleunigung, der Weg und/oder die Geschwindigkeit, des Zahnbehandlungsmittels gegenüber einem sich mit dem Kopf der behandelten Person mitbewegenden Bezugssystems, erfasst werden, den Schritt des Definierens eines weiteren dreidimensionales Bezugssystem durch eine Prozessoreinrichtung, wobei sich das weitere Bezugssystem bei einer Bewegung des Zahnbehandlungsmittels im Raum zumindest zeitweise und bevorzugt stets mit dem Zahnbehandlungsmittel mitbewegt, wobei das weitere Bezugssystem durch charakteristische Körperpunkte der Hand, Körperlinien der Hand und/oder Körperoberflächen der Hand, mit der das Zahnbehandlungsmittel geführt wird, gebildet wird, des Schrittes des Bereitstellens der erfassten Daten an eine Prozessoreinrichtung zur Bestimmung des Bewegungsmusters und des Schrittes der Bestimmung des Bewegungsmusters.

Bevorzugt wird das Zahnbehandlungsmittel zumindest zeitweise dreidimensional, d.h. in X-, Y- und Z- Richtung, bewegt und die Bewegungsparameter werden besonders bevorzugt richtungs- und/oder orientierungsabhängig erfasst. Die X-Y-Ebene ist eine Ebene, die sich in X-Richtung und in Y-Richtung erstreckt, analog erstrecken sich mögliche andere Ebenen, wobei die X-/Y-/Z-Achsen jeweils rechtwinklig zueinander stehen.

Alternativ oder zusätzlich kann sich die vorliegende Erfindung auf ein Verfahren zum zumindest teilweisen Bestimmen eines sich bei einer Zahnbehandlung, insbesondere Zahnreinigung, ergebenden, insbesondere personenbezogenen, Bewegungsmusters eines Zahnbehandlungsmittels, insbesondere einer Zahnbürste, beziehen, wobei das Verfahren bevorzugt zumindest den Schritt des optischen Erfassens von Daten, insbesondere mittels mindestens einer Kamera, zu mindestens einem Bewegungsparameter, insbesondere der Bewegungsrichtung oder -rotation, der Beschleunigung, des zurückgelegten Wegs und/oder der Geschwindigkeit, eines Zahnbehandlungsmittels und den Schritt der bildlichen Separation des Zahnbehandlungsmittels, insbesondere der das Zahnbehandlungsmittel führenden Hand, von mindestens einem weiteren durch die optische Erfassungseinrichtung erfassten Bildbestandteil mittels einer Datenverarbeitungseinrichtung und den Schritte des Bestimmens von mindestens einem zu dem Bewegungsparameter korrespondierenden Bewegungsmuster aufweist.

Diese Lösung ist vorteilhaft, da die Datenerfassung sowohl orts- als auch zeitungebunden stattfindet und somit auch zuhause bei Privatpersonen jederzeit d.h. uhrzeitunabhängig eine Überwachung und objektive Beurteilung der Zahnbehandlung erfolgen kann. Die erfassten Daten sind derart, insbesondere in Echtzeit, aufzubereiten bzw. werden bevorzugt in Echtzeit derart aufbereitet, dass z.B. von einem behandelnden Zahnarzt oder der Privatperson bzw. dem Patienten selbst das Bewegungsmuster analysierbar ist, wodurch individuelle Anpassungen der bei der Zahnbehandlung erfolgenden Bewegungsabläufe erfolgen können. Die so erhobenen Daten können nicht nur individuell zur akuten Optimierung der Bewegungsmuster genutzt werden, sondern können auch in größerer Skalierung z.B. von Krankenkassen und/oder Forschungseinrichtungen verwendet werden. Durch Übermittlung der Daten auf eine zentrale Auswertungsstation wird z.B. zum einen die Möglichkeit eröffnet, die erzeugten Rohdaten zentralisiert mit adäquater Rechenleistung zu verarbeiten, mit optimalen Bewegungsschemata zu vergleichen und an die lokalen Endgeräte zu senden, zum anderen wird aber auch zusätzlich oder alternativ die Bereitsteilung der Daten für groß angelegte klinische Studien zu Forschungszwecken oder für die kommerzielle statistische Nutzung (z.B. für Krankenkassen) ermöglicht.

Gemäß einer weiteren bevorzugten Ausführungsform der vorliegenden Erfindung wird ein Verfahren zur Bestimmung von mindestens einer Bewegungscharakteristik bereitgestellt. Das Verfahren umfasst bevorzugt mindestens den Schritt, des vielfachen Ausführens des zuvor genannten Verfahrens, insbesondere auf verschiedenen Endgeräten zur Erfassung von Bewegungsmustern unterschiedlicher Personen und bevorzugt deren statistische Auswertung, wobei zusätzlich oder alternativ auch denkbar ist, dass eine Bewegungscharakteristik für eine Person erzeugbar bzw. bestimmbar ist, und den Schritt des Bestimmens mindestens einer übereinstimmenden Eigenschaft, insbesondere eines Bewegungsparameters und/oder der Zahnbehandlungsdauer, der bestimmten Bewegungsmuster oder der erfassten Daten. Diese Ausführungsform ist vorteilhaft, da aus den Bewegungsmustern individueller Personen jeweils eine oder mehrere Bewegungscharakteristika ableitbar sind, die repräsentativ für das Zahnbehandlungsverhalten der jeweiligen Person sind. Es ist jedoch zusätzlich oder alternativ denkbar, dass die Bewegungsmuster oder Bewegungscharakteristika individueller Personen aufgrund übereinstimmender Merkmale zu gruppieren sind bzw. gruppiert werden. Weiterhin ist denkbar, dass ein Schritt des Abgleichens der bestimmten Bewegungsmuster oder der erfassten Daten mit charakteristischen Standarddaten erfolgt, wobei diese in einer Datenbank bereitgestellt werden, wobei die Datenbank zumindest teilweise lokal auf einem mobilen Endgerät vorgehalten wird und/oder zumindest teilweise auf einem Server vorgehalten wird.

Weiterhin ist denkbar, dass während der Benutzung des Gerätes ein Schritt des Ausgebens, insbesondere optischen, haptischen und/oder akustischen Ausgebens, zur Information über Korrektur-, Standard- und/oder Idealbewegungen, insbesondere in Abhängigkeit von dem aktuell, d.h. z.B. in Echtzeit, bestimmten Bewegungsmuster oder in Abhängigkeit eines bereits bestimmten Bewegungsmusters oder einer Bewegungscharakteristik vorgesehen ist. Optisches Ausgeben bedeutet hierbei bevorzugt das bildliche Darstellen der Korrektur-, Standard- und/oder Idealbewegungen auf einem Bildschirm oder einer Projektionsfläche, wobei denkbar ist, dass bildliches Ausgeben als Darstellung einzelner Bilder und/oder einer animierten Filmdarstellung verstanden werden kann. Akustisches Ausgeben beschreibt hierbei bevorzugt das Ausgeben bestimmter Töne und/oder das Ausgeben von Sprachanweisungen. Haptisches Ausgeben kann z.B. als definierte Ausgabe von Vibrationen verstanden werden. Insbesondere hinsichtlich der optischen Ausgabe sind bevorzugt die Korrektur-, Standard- und/oder Idealbewegungen neben, über und/oder in Abgleich zu dem tatsächlich erfassten Bewegungsmuster oder zu den tatsächlich erfassten Bewegungsmustern ausgebbar. Korrekturbewegungen können hierbei z.B. einen Vorschlag für eine Bewegungsänderung bzw. -anpassung gegenüber dem erfassten Bewegungsmuster anzeigen. Standardbewegungen können z.B. die durchschnittlichen Bewegungen einer definierten Gruppe an Personen oder die aus klinischen Studien als optimal bekannten Bewegungen sein. Idealbewegungen können z.B. Bewegungen sein, die äußerst schonend, reinigend und/oder zeiteffizient, etc. sind. Gemäß einer weiteren bevorzugten Ausführungsform der vorliegenden Erfindung wird ein Verfahren zum Überwachen von Zahnbehandlungsbewegungen und zum Ausgeben von Korrekturparametern zum Anpassen der Zahnbehandlungsbewegungen bereitgestellt. Das Verfahren umfasst das Verfahren 2um zumindest teilweisen Bestimmen eines sich bei einer Zahnbehandlung, insbesondere Zahnreinigung, ergebenden Bewegungsmusters eines Zahnbe ha ndlungsm Ittels, insbesondere einer Zahnbürste, mit den Schritten: Bewegen des Zahnbehandlungsmittel zum Behandeln von Oberflächenanteilen der Zähne zumindest in einer X-/Y-Ebene, wobei mittels einer bevorzugt optischen Detektionseinrichtung, insbesondere einer Sensoreinrichtung, Daten bezüglich mindestens eines Bewegungsparameters des Zahnbehandlungsmittels, insbesondere die Bewegungsrichtung oder -rotation, die Beschleunigung, der Weg und/oder die Geschwindigkeit, erfasst werden, und Bereitstellen der erfassten Daten an eine Prozessoreinrichtung oder an ein System zur Bestimmung des Bewegungsmusters und die Schritte Abgleichen der erfassten Bewegungsmuster mit in einer Datenbank vorgehaltenen Bewegungsmustern, Ableiten oder Bestimmen von Korrekturparametern, insbesondere mindestens einer Bewegungsrichtung, zur Anpassung der Bewegung des Zahnbehandlungsmittels in Abhängigkeit von den abgeglichenen Bewegungsmustern, und Ausgeben einer Information mittels einer Ausgabeeinrichtung in Abhängigkeit von den abgeleiteten oder bestimmten Korrekturparametern. Dieses Verfahren ist vorteilhaft, da es einer eine Zahnbehandlung, insbesondere Zahnreinigung, durchführenden Person bevorzugt unmittelbar eine Rückmeldung bzw. Korrekturinformation zum Bewegungsablauf ausgibt bzw. einer die Zahnbehandlung durchführenden Person, insbesondere in Echtzeit, Korrekturinformationen zur Korrektur bzw. Anpassung des Bewegungsablaufs ausgebbar sind, sollte sie das Zahnbehandlungsmittel mit einer, insbesondere softwaretechnisch, als ungeeignet bewerteten Bewegung oder einem als ungeeignet bewertetem Bewegungsmuster führen.
Diese Ausführungsform ist vorteilhaft, da so z.B. Kindern die hinsichtlich der Bewegungsabläufe bei der Zahnbehandlung, insbesondere Zahnreinigung, identifizierten geeigneten Bewegungsmuster, insbesondere überwacht, gelehrt werden können. Eine optische Ausgabeeinrichtung, wie z.B. das Display eines Mobiltelefons, einer Uhr, eines Armbands, einer Spieiekonsole oder eines TabletPCs, kann dabei ein Bild oder ein Video ausgeben, das dem Kind die bevorzugt optimalen Bewegungsmuster, insbesondere die in einer lokalen Speichereinrichtung und/oder auf einem Server und/oder in einer lokalen oder internetbasierten Datenbank in Datenform vorgehaltenen Bewegungsmuster, optisch anzeigt bzw. ausgibt. Mittels einer optischen Detektionseinrichtung oder optischen Erfassungseinrichtung, insbesondere einer Kamera, die bevorzugt mit der Ausgabeeinrichtung signaltechnisch gekoppelt ist und bevorzugt im selben Gehäuse, wie die Ausgabeeinrichtung, untergebracht ist, sind die Bewegungen des Kindes erfassbar und somit überwachbar. Sollte das Kind von den angezeigten Bewegungen bzw. Bewegungsmustern abweichen, dann ist bevorzugt ein Signal ausgebbar, dass dem Kind die Abweichung verdeutlicht. Bevorzugt sind die Detektionseinrichtung und die Ausgabeeinrichtung, insbesondere zum optischen und/oder haptischen und/oder akustischen Ausgeben von Signalen und/oder Anweisungen, Bestandteil einer einzigen Vorrichtung, insbesondere einer mobilen Vorrichtung. Die Vorrichtung ist hierbei bevorzugt als portable Prozessoreinrichtung, insbesondere als Mobiltelefon oder TabletPC, ausgebildet. Gemäß einer weiteren bevorzugten Ausführungsform der vorliegenden Erfindung erfolgt bevorzugt eine Registrierung der zu behandelnden Zähne, wobei die Registrierung die optische Erfassung mittels der Detektionseinrichtung oder eine manuelle Erfassung mittels einer Eingabemaske umfasst, wobei die Anzahl und/oder die Lage und/oder die Ausrichtung und/oder Defekte von zumindest einzelnen Zähnen erfasst werden, und wobei die Ableitung oder Bestimmung der Korrekturparameter bevorzugt unter Berücksichtigung der registrierten Zähne erfolgt. Die Eingabemaske kann hierbei z.B. über eine Anzeigeeinrichtung eines Endgeräts, insbesondere des Endgeräts das auch die Detektionseinrichtung aufweist, visualisiert werden. Bevorzugt kann mittels einer Eingabeeinrichtung, die bevorzugt Teil der Anzeigeeinrichtung sein kann, von einer Person angegeben werden, welche Anzahl an Zähnen vorliegt und/oder welche Lage und/oder welche Ausrichtung und/oder welche Defekte einzelne, mehrere oder alle Zähne aufweisen. Bevorzugt erfolgt die Ableitung oder Bestimmung der Korrekturparameter unter Berücksichtigung der registrierten Zähne. Die Registrierung der Zähne kann hierbei z.B. mittels der, insbesondere optischen, Detektionseinrichtung, insbesondere einer Kamera, durchgeführt werden, d.h. es wird bevorzugt mindestens ein Bild des Gebisses gemacht, wobei softwaretechnisch das Bild ausgewertet wird und z.B. die Anzahl, Ausrichtung und/oder Lage und/oder Defekte der Zähne erfasst wird. Es ist somit bevorzugt ebenfalls möglich Leerstellen im Gebiss zu erfassen.
Zusätzlich oder alternativ ist denkbar, dass die Anzahl, Ausrichtung und/oder Lage und/oder Defekte der Zähne manuell, insbesondere über eine Eingabemaske, erfassbar sind. Zusätzlich oder alternativ sind das Winkelmerkmal und/oder Versiegelungen und/oder Kronen und/oder Inlays bzw. Plomben, eines oder mehrere Zähne, insbesondere mittels der optischen Erfassungseinrichtung, erfassbar. Die Lage der Zähne beschreibt bevorzugt die Position der Zähne bzw. um welchen Zahn es sich bei dem jeweiligen Zahn handelt (z.B. Inzisoren, Canine, Premolaren, Molaren). Weiterhin ist denkbar, dass Eigenschaften der einzelnen Zähne oder Relationen zwischen den einzelnen Zähnen erfassbar sind.
Als Defekte werden bevorzugt Zahnfleischrückgang und/oder Verfärbungen und/oder Zahnstein und/oder Risse und/oder Abbruche und/oder Löcher und/oder Zahnstein etc. verstanden.
Weiterhin ist bevorzugt denkbar, dass einmalig oder wiederholt bzw. mehrfach ein Kalibrierungsschritt durchgeführt wird. Als Kalibrierung wird hierbei bevorzugt die Bestimmung der konkreten bzw.
persönlichen Eigenschaften des Körpers, insbesondere der Größe oder des Abstands des Kopfes zur Detektionseinrichtung oder einem Referenzpunkt bzw. der Größe oder des Abstands eines Bestandteils des Kopfes zur Detektionseinrichtung oder zu einem Referenzpunkt, verstanden. Besonders bevorzugt werden bei der Kalibrierung von der optischen Erfassungseinrichtung mehrere körperfeste Punkte bzw. Oberflächenpunkte erfasst, wobei bevorzugt ein oder mehrere Frontzähne und/oder der sich in Zahnlängsrichtung erstreckende Kontaktbereich bzw. Grenzbereich zwischen den Frontzähnen erfasst wird. Es handelt sich hierbei bevorzugt um die zentralen Schneidezähne, insbesondere des Oberkiefers, wobei denkbar ist, dass auch oder alternativ die zentralen Schneidezähne des Unterkiefers erfasst werden. Die Erfassung der Position der Schneidezähne des Oberkiefers und/oder des Grenzbereichs zwischen diesen Schneidezähnen ist oftmals bei hochgezogener Oberlippe möglich, was jedoch beim Zähneputzen nicht immer sichergestellt sein kann. Dennoch bietet die Erfassung der Position der Schneidezähne und/oder des Grenzbereichs zwischen den Schneidezähnen in Relation zu weiteren anthropometrischen Punkten der Gesichtsoberfläche, insbesondere der Nase, Wangen und Stirnregion, Falten, insbesondere Stirnfalten, oder den Pupillen, eine Möglichkeit ein äußerst präzises
Koordinatensystem bzw. Bezugssystem am Kopf einer Person zu definieren. Dieses soll bzw. kann für klinische Studien vorzugsweise mit etablierten zahnmedizinischen Koordinatendefinitionen wie bspw. der Frankfurter Horizontalen; der Camperschen Ebene oder der Okklusalebene in Bezug gesetzt werden. Weiterhin kann ein so definiertes Koordinatensystem zusätzlich an genau oder mindestens eine, zwei, drei weitere Körperpunkte oder an mehrere Körperpunkte, insbesondere die Nase, das Kinn, die Stirn, ein Ohr oder zwei Ohren, etc. gekoppelt werden. Dies ist vorteilhaft, da zum einen das einmal definierte Koordinatensystem unabhängig von der Stellung der Lippen stets erfassbar bleibt, wodurch die Position der Zähne stets bekannt ist, und zum anderen die Form des Körperteils, z.B. der Nase, irrelevant ist. Die eventuell auftretende Weichteildeformierung bei der Mundöffnung soll bzw. kann durch entsprechende Korrekturrechnungen kompensiert werden um die Genauigkeit der Methode weiter zu erhöhen.

Gemäß einer weiteren bevorzugten Ausführungsform der vorliegenden Erfindung erfolgt die optische Erfassung eines Objekts, wobei zumindest die Erstreckung des Objekts in einer Dimension, d.h. in der Länge und/oder in der Breite und/oder in der Höhe und/oder im Umfang, bekannt ist, und die Erstreckung des Objekts in der bekannten Dimension bzw. in den bekannten Dimensionen wird bevorzugt zur Bestimmung von Dimensionsparametern verwendet, um z.B. die Länge des bei einer Zahnbehandlung vom Zahnbehandlungsmittels zurückgelegten Wegs und/oder die Dimensionen der Zähne, insbesondere der sichtbaren Anteile der Zähne, etc. zu erfassen. Das Objekt ist hierbei bevorzugt die Person, die das Zahnbehandlungsmittel verwendet, wobei die bekannte Dimension der Person bevorzugt deren Größe ist. Zusätzlich oder alternativ ist denkbar, dass das Objekt das Zahnbehandlungsmittel, insbesondere die Zahnbürste, ist, wobei die bekannte Dimension der Zahnbürste bevorzugt die Länge und/oder die Breite und/oder die Höhe und/oder der Umfang ist. Zusätzlich oder alternativ ist denkbar, dass das Objekt ein Element ist, dessen Abmessungen im Wesentlichen überall auf der Welt identisch sind, wie z.B. eine CD, DVD oder Kreditkarte. Bevorzugt sind Daten zu den Eigenschaften des Zahnbehandlungsmittels mittels eines Identifikationsmittels (vgl. spätere Ausführungen) erfassbar.
Weiterhin ist denkbar, dass in oder an dem Zahnbehandlungsmittel oder zum in Kontakt bringen mit dem Zahnbehandlungsmittel eine Sensoreinrichtung, insbesondere ein Drucksensor, zur Erfassung des mittels des Zahnbehandlungsmittels auf den Mund bzw. die Zähne und/oder das Zahnfletsch ausgeübten Anpressdrucks vorgesehen ist. Alternativ oder zusätzlich ist vorstellbar, dass die Druckerfassung mittels der optischen Erfassungseinrichtung bewirkt wird. Wobei eine Biegung des Zahnbehandlungsmittels erfasst wird und in Abhängigkeit von bekannten Festigkeitswerten des Zahnbehandlungsmittels gesetzt wird. Die Festigkeitswerte können dabei in einer Datenbank hinterlegt sein oder hinterlegbar sein. Weiterhin ist denkbar, dass das Zahnbehandlungsmittel direkt oder die Verpackung desselbigen (z.B. Barcode oder QR-Code) optisch erfasst und identifiziert wird. Die Identifikation des Zahnbehandlungsmittels ermöglicht dabei, dass die zu dem konkreten Zahnbehandlungsmittel gehörenden Informationen bzgl. seiner Eigenschaften, insbesondere die Festigkeitswerte, selbsttätig für eine Prozessoreinrichtung hinterlegt oder abgerufen werden. Weiterhin ist denkbar, dass die Informationen bzgl. der Eigenschaften eines Zahnbehandlungsmittels auch manuell in einer Datenbank, insbesondere eines mobilen Endgeräts, verfügbar gemacht werden bzw. hinterlegbar bzw. registrierbar sind. Bevorzugt sind die Informationen bzgl. der Eigenschaften in Datenform lokal auf dem mobilen Endgerät oder auf einem Server abspeicherbar. Gemäß einer weiteren bevorzugten Ausführungsform der vorliegenden Erfindung erfolgt somit bevorzugt eine Bestimmung eines bei der Zahnbehandlung, insbesondere einer Zahnreinigung, mittels eines Zahnbehandlungsmittels, insbesondere einer Zahnbürste, auf die Zähne oder das Zahnfleisch der die Zahnreinigung erfahrenden Person ausgeübten Drucks, wobei eine optische Erfassung des Zahnbehandlungsmittels, insbesondere mittelbar oder unmittelbar, mittels der optischen Detektionseinrichtung erfolgt, wobei durch die Detektionseinrichtung Bildinformationen erfasst bzw. Bilddaten erzeugt werden und mittels der Bildinformationen bzw. der Bilddaten eine, insbesondere elastische, Verformung, insbesondere Durchbiegung, des Zahnbehandlungsmittels bestimmt wird, wobei der Druck zumindest in Abhängigkeit der Verformung des Zahnbehandlungsmittels und bevorzugt weiterer Daten, die Eigenschaften des Zahnbehandlungsmittels umfassen, bestimmt wird. Weiterhin ist denkbar, dass in Abhängigkeit des ermittelten Drucks über eine Ausgabeeinrichtung ein Signal oder eine Information ausgebbar ist. Bevorzugt repräsentiert das Signal oder die Information eine Handlungsempfehlung an den Benutzer des Zahnbehandlungsmittels, insbesondere an die Person, welche die Zahnbehandlung erfährt. Die Erfassung des Drucks bzw. die Ausgabe der in Abhängigkeit des detektierten Drucks auszugebenden Signale und/oder Informationen erfolgt bevorzugt in Echtzeit. Besonders bevorzugt erfolgt die Ausgabe der Signale und/oder Informationen über eine optische Ausgabeeinrichtung. Bevorzugt ist die optische Ausgabeeinrichtung Teil einer Vorrichtung, insbesondere des Endgeräts, zu der ebenfalls die optische Detektionseinrichtung gehört. Besonders bevorzugt werden die Signale und/oder Informationen zum Druck zeitgleich und/oder über dasselbe Ausgabegerät an dieselbe Person kommuniziert, mit dem ebenfalls Korrekturparametern zum Anpassen der Zahnbehandlungsbewegungen an eine Person, insbesondere die Person, die eine Zahnbehandlung erfährt und/oder durchführt, kommuniziert werden. Bei den Daten zu den Eigenschaften des Zahnbehandlungsmittels kann es sich um Materialeigenschaften und/oder Festigkeitswerte und/oder Abmessungen und/oder Biegesteifigkeiten und/oder Fabrikat und/oder Alter und/oder Benutzungshäufigkeit, insbesondere Benutzungsdauer, etc. handeln.
Bevorzugt können gemäß einer weiteren bevorzugten Ausführungsform der vorliegenden Erfindung mittels der optischen Detektionseinrichtung mindestens drei bevorzugt vorgegebene Punkte, insbesondere Oberflächenpunkte, eines Körpers, insbesondere eines Kopfes, einer Person, insbesondere bzgl. der die Bewegungsmuster erfasst werden, erfasst werden, wobei bevorzugt mindestens zwei Punkte mit einer Linie verbindbar sind bzw. virtuell verbindbar sind bzw. gedacht verbindbar sind und mindesten ein dritter Punkt nicht auf dieser Linie liegt, wobei durch die mindesten drei erfassten Punkte das Bezugssystem definiert wird und wobei eine Bewegung des Zahnbehandlungsmittels gegenüber diesem Bezugssystem erfasst wird oder wobei mittels der optischen Detektionseinrichtung mindestens drei bevorzugt vorgegebene Punkte des Zahnbehandlungsmittels, insbesondere einer Zahnbürste, erfasst werden, wobei mindestens zwei Punkte mit einer Linie verbindbar sind und mindesten ein dritter Punkt nicht auf dieser Linie liegt, wobei durch die mindesten drei erfassten Punkte das Bezugssystem definiert wird und wobei eine Bewegung der Person, insbesondere des Kopfes, gegenüber diesem Bezugssystem erfasst wird.
Gemäß einer weiteren bevorzugten Ausführungsform der vorliegenden Erfindung werden in Abhängigkeit von einer Bewegung des Körpers, insbesondere des Kopfes, der Person, bzgl. der die Bewegungsmuster erfasst werden, gegenüber der optischen Detektionseinrichtung unterschiedliche Punkte des Körpers, insbesondere des Kopfes, der Person zur Definition des Bezugssystems erfasst. Diese Ausführungsform ist vorteilhaft, da der Kopf der Person beim Durchführen des Verfahrens nicht vollständig unbewegt sein muss, sondern sich bevorzugt bewegen kann und dennoch eine genaue Erfassung der Bewegungsmuster möglich ist. Sollte z.B. infolge einer Kopfdrehung der Person ein zur Definition des Bezugssystems erfasster Oberflächenpunkt nicht weiter erfassbar sein, wird bevorzugt bereits bevor der Oberflächenpunkt nicht mehr erfassbar ist, ein anderer körperfester bzw. kopffester Punkt bzw. Oberflächenpunkt oder mehrere andere körperfeste bzw. kopffeste Punkte bzw. Oberflächenpunkte erfasst, mittels dem/denen das Bezugssystem weiter aufrecht erhalten wird. Als körperfeste oder kopffeste Punkte bzw. Oberflächenpunkte können z.B. neben charakteristischen Formen, wie z.B. der Nasenspitze, Hautverfärbungen, insbesondere Pigmentsörungen, Pigmentnävus, Narben, Körperschmuck, insbesondere Tattoos, die Augenhöhlen, die Schneidezähne, insbesondere des Oberkiefers, auch Punkte von am Körper, insbesondere am Kopf, angebrachten Einrichtungen, wie z.B. Kleidung, Mütze, Brille, erfasst werden.
Gemäß einer weiteren bevorzugten Ausführungsform der vorliegenden Erfindung ist die Prozessorvorrichtung ein mobiles Endgerät (z.B. Mobiltelefon oder Tablet PC), das die erfassten Daten über das Internet an eine Servereinrichtung zur weiteren Aufbereitung sendet und/oder über das Internet Daten zur Ausgabe mittels einer Ausgabeeinrichtung des Gerätes empfängt, wobei die Daten Informationen bezüglich Bewegungs Parametern des Zahnbehandlungsmittels umfassen. Weiterhin wird die zuvor genannte Aufgabe durch ein System zum zumindest teilweisen bzw. zumindest teilweise indirekten Bestimmen eines sich bei einer Zahnbehandlung, insbesondere Zahnreinigung, ergebenden personenbezogenen und bevorzugt dynamischen Bewegungsmusters eines Zahnbehandlungsmittels, insbesondere einer Zahnbürste, gelöst. Ein dynamisches Bewegungsmuster ist hierbei bevorzugt als ein Bewegungsmuster zu verstehen, das zumindest teilweise durch Geschwindigkeitsinformationen und/oder Beschleunigungsinformationen des Zahnbehandlungsmittels gegenüber einem Behandlungsbereich definiert ist. Das System umfasst bevorzugt mindestens eine Detektionseinrichtung, insbesondere eine Sensoreinrichtung oder eine optische Erfassungseinrichtung, zum Erfassen von Daten zu mindestens einem Bewegungsparameter, insbesondere die Bewegungsrichtung, die Beschleunigung, der zurückgelegte Weg und/oder die Geschwindigkeit, des Zahnbehandlungsmittels, und mindestens oder genau eine Datenverarbeitungseinrichtung zur Erzeugung von Bewegungsmustern anhand der erfassten Daten. Es ist hierbei denkbar, dass die Datenverarbeitungseinrichtung bevorzugt eine mit dem Internet zumindest zeitweise verbindbare Einrichtung, insbesondere ein Computer, eine Digitalkamera, eine Spielkonsole, ein Tabletcomputer, ein Laptop, eine Armbanduhr (z.B. Smartwatch), ein Fernsehgerät und/oder ein Mobiltelefon, ist. Weiterhin ist denkbar, dass die Datenverarbeitungseinrichtung die Bewegungsmuster teilweise oder vollständig erzeugt. Besonders bevorzugt übermittelt die Datenverarbeitungseinrichtung die vollständig oder teilweise erzeugten Bewegungsmuster an eine Servereinrichtung, die bevorzugt von einer Vielzahl an Datenverarbeitungseinrichtungen personenbezogene Bewegungsmusters von Zahnbehandlungsmitteln erhält. Die Datenübermittlung kann z.B. in Abhängigkeit von einem Energie-, Verbindungs-, Zeit-, Datenvolumen- und/oder Zahnbehandlungswiederholungskriterium erfolgen, wobei das Zahnbehandlungswiederholungskriterium bevorzugt eine konkrete Anzahl an Zahnbehandlungen angibt. Das Verbindungskriterium gibt bevorzugt die Art und/oder Qualität der Internetverbindung an, z. B. ob es sich um ein Mobilfunktarif oder ein lokales Netzwerk, wie z. B. ein WLAN, handelt. Das Energiekriterium gibt bevorzugt einen Ladezustand eines durch einen Akkumulator mit Energie, insbesondere Strom, versorgten Endgeräts, insbesondere mobilen Endgeräts, wieder. Diese kriteriumsabhängige Übermittlung der teilweise oder vollständig erzeugten Bewegungsmuster ist vorteilhaft, da die Berechnung der Bewegungsmuster zunächst auf der Seite der Datenverarbeitungseinrichtung, d. h. endgeräteseitig, erfolgen kann und somit die Menge an Datentransfer zwischen den einzelnen Endgeräten und der Servereinrichtung relativ gering gehalten werden kann. Ferner ist dies vorteilhaft, da die Servereinrichtung eine deutlich geringere Rechenleistung aufweisen muss, wenn sie bereits teilweise oder vollständig aufbereitete Bewegungsmuster empfängt und diese weiterverarbeiten kann. Die Servereinrichtung muss dann bevorzugt nicht erst noch die vollständige Erzeugung der Bewegungsmuster aus den Rohdaten bewirken bzw. berechnen. Alternativ ist jedoch auch denkbar, dass die von der Detektionseinrichtung erfassten Daten zur Weiterverarbeitung an die Servereinrichtung weitergeleitet werden. Die Weiterleitung kann dabei unmittelbar nach der Erfassung oder in Abhängigkeit bestimmter Kriterien erfolgen. Die Kriterien können dabei z. B. ein Energie-, Verbindungs-, Zeit-, Datenvolumen- und/oder Zahnbehandlungswiederholungskriterium sein.
Bevorzugt kann die Servereinrichtung in dieser Fallgestaltung als Datenverarbeitungseinrichtung angesehen werden, d. h. endnutzerseitig bzw. im Bereich der Detektionseinrichtung ist nicht zwingend eine Datenverarbeitungseinrichtung erforderlich, die eine Aufbereitung der erfassten Daten bewirkt.
Bevorzugt ist jedoch eine Prozessoreinrichtung, insbesondere ein Mobiltelefon, vorgesehen, die zumindest eine Weiterleitung der Daten hin zu der Servereinrichtung bewirkt. Die Servereinrichtung hat in einer besonders bevorzugten Ausführungsform Zugriff auf klinisch erhobene Informationen über den Zahngesundheitszustand, die Anzahl an Zahnarztbesuche je Jahr und/oder die Behandlungshistorie, die verwendeten Zahnreinigungsmittel etc. von konkreten Personen auf und verknüpft diese Informationen mit den erfassten Bewegungsmustern der jeweiligen das Gerät verwendenden Person. Die erfassten Bewegungsparameter einer konkreten Person sind bevorzugt mittels einer Personalisierungsinstanz, wie z. B. einer Namensabfrage, einer Passwortabfrage, einer Telefonnummer einer Emailadresse oder einem Pseudonym, dieser konkreten Person zuordenbar. Diese Ausführungsform ist vorteilhaft, da mittels den von der Servereinrichtung erzeugten Daten bestehend aus dem Zahngesundheitszustand, der Anzahl an Zahnarztbesuche je Jahr und/oder der Behandlungshistorie und den erfassten Bewegungsparametern bzw. den Bewegungsmustern eindeutige Kontrollen und Überwachungen vornehmbar sind und Lehren zur weiteren Verbesserung der Zahnbehandlungstechnik ziehbar sind.
Ein bevorzugt in dem erfindungsgemäßen System einsetzbares bzw. verwendbares Zahnbehandlungsmittel, insbesondere eine Zahnbürste, Munddusche, Gasstrahler, insbesondere Luftstrahler, Sandstrahler, Bohrer, Ultraschallreiniger, etc., umfasst bevorzugt mindestens eine Leitstützstruktur, wobei an der Leitstützstruktur eine Behandlungseinrichtung zum zumindest mittelbaren Inkontaktbringen mit einer Zahnoberfläche und ein Kontaktbereich zum Halten des Zahnbehandlungsmittels angeordnet oder ausgebildet sind, wobei eine Detektionseinrichtung, insbesondere ein Positions-, Geschwindigkeits- Beschleunigungs- und/oder Rotationssensor, zur Erfassung von Bewegungsparametern zumindest während einer Verwendung des Zahnbehandlungsmittels mit der Leitstützstruktur körperlich verbunden ist. Es ist hierbei denkbar, dass die Detektionseinrichtung an ein beliebiges Zahnbehandlungsmittel ankoppelbar bzw. angekoppelt ist. Weiterhin ist denkbar, insbesondere im Falle von elektrischen Zahnbürsten, dass die Detektionseinrichtung fest bzw. dauerhaft mit der elektrischen Zahnbürste verbunden ist. Gemäß einer weiteren bevorzugten Ausführungsform der vorliegenden Erfindung weist das Zahnbehandlungsmittel eine Kommunikationsschnittstelle zum Übertragen der erfassten Daten, insbesondere in roher oder aufbereiteter Form, an eine Datenverarbeitungseinrichtung auf, wobei die Kommunikationsschnittstelle besonders bevorzugt derart gestaltet ist, dass die Daten kabellos übertragbar sind.
Gemäß einer weiteren bevorzugten Ausführungsform der vorliegenden Erfindung weist das System eine Prozessorvorrichtung, insbesondere ein mobiles Endgerät, wie z.B. ein Mobiltelefon, auf, das bevorzugt mindestens eine optische Erfassungseinrichtung, insbesondere eine Kamera, zum Erfassen von Daten von zu mindestens einem Bewegungsparameter, insbesondere der Bewegungsrichtung oder -rotation, der Beschleunigung, des zurückgelegten Wegs und/oder der Geschwindigkeit, eines Zahnbehandlungsmittels und eine Datenverarbeitungseinrichtung zur insbesondere bildlichen Separation des Zahnbehandlungsmittels von mindestens einem weiteren durch die optische Erfassungseinrichtung erfassten Bildbestandteil und zum Bestimmen von mindestens einem zu dem Bewegungsparameter korrespondierenden Bewegungsmuster aufweist.
Die optische Erfassungseinrichtung wird bevorzugt in dem System als Detektionseinrichtung zum zumindest teilweisen Bestimmen eines sich bei einer Zahnbehandlung, insbesondere Zahnreinigung, ergebenden personenbezogenen Bewegungsmusters verwendet. Im Falle einer Detektionseinrichtung, die bevorzugt als optische Detektionseinrichtung oder optische Erfassungseinrichtung ausgebildet ist, ist denkbar, dass das Zahnbehandlungsmittel mit einer optisch charakteristischen Form versehen ist, die bevorzugt einem Koordinatenkreuz entspricht bzw. dessen Funktionalität übernimmt, um zusätzlich eine räumliche Erfassung eines konkreten Bewegungsparameters des Zahnbehandlungsmittels beim Einsatz von genau oder mindestens einer Kamera zu ermöglichen. Zusätzlich oder alternativ ist jedoch auch denkbar, dass das Zahnbehandlungsmittel mit einer oder mehreren Sensoreinrichtungen ausgestattet ist und die Datenverarbeitungseinrichtung zur Bestimmung des Bewegungsparameters die Sensordaten und die Daten der optischen Erfassungseinrichtung verwendet.
Weiterhin ist ebenfalls denkbar, dass das Zahnbehandlungsmittel auch mit einer oder mehreren Sensoreinrichtungen ausgestattet ist, wenn die Prozessoreinrichtung oder das System mindestens oder genau eine, zwei oder mehrere Kameras, insbesondere 3 Kameras, aufweist. Die Datenverarbeitungseinrichtung kann in diesem Falle Daten einzelner oder mehrerer oder aller Kameras und/oder Sensoren für die Bestimmung des Bewegungsparameters heranziehen.
Gemäß einer weiteren bevorzugten Ausführungsform der vorliegenden Erfindung weist die optische Erfassungseinrichtung mindestens eine, insbesondere genau oder mindestens zwei, Kameras zur dreidimensionalen Erfassung des mindestens einen Bewegungsparameters eines Zahnbehandlungsmittels auf, wobei die Prozessoreinrichtung ein dreidimensionales Koordinatensystem bzw. Bezugssystem definiert, das sich bei einer Bewegung des Zahnbehandlungsmittels im Raum mit dem Zahnbehandlungsmittel mitbewegt. Das Koordinaten- bzw. Bezugssystem kann dabei durch charakteristische anthropometrische Körperpunkte, Körperlinien und/oder Körperoberflächen eines Körperteils einer Person, insbesondere der Hand, mit der das Zahnbehandlungsmittel geführt wird, gebildet werden. Es kann zusätzlich jedoch auch durch Oberflächenpunkte, -linien und/oder - flächen des Zahnbehandlungsmittels gebildet werden. Die zweite Kamera ist dabei bevorzugt fixiert in Relation zur ersten Kamera angeordnet ist, so dass eine einmalige Kalibrierung des Detektionssystems vor der Verwendung erfolgen kann. Bevorzugt ist ebenfalls denkbar, dass die mindestens zwei Kameras gegeneinander beweglich sind. Bevorzugt handelt es sich bei den mindestens zwei Kameras um mindestens, genau oder maximal 2, 3, 4, 5 oder 6 Kameras.
Gemäß einer weiteren bevorzugten Ausführungsform der vorliegenden Erfindung erfasst die optische Erfassungseinrichtung einen Anteil des Kopfes bzw. mindestens drei Punkte auf der Oberfläche des Kopfes bzw. mindestens drei körperfeste Punkte, die sich bei einer Bewegung der Person, insbesondere des Kopfes, mit der Person, insbesondere mit dem Kopf, mitbewegen und legt ein dreidimensionales Koordinatensystem bzw. Bezugssystem in dem erfassten Anteil des Kopfs bzw. mittels der Punkte fest, wobei eine Bewegung des Zahnbehandlungsmittels in Abhängigkeit des festgelegten Koordinatensystems des Kopfes bestimmt wird oder eine Bewegung des erfassten Anteils des Kopfes in Abhängigkeit des Koordinatensystems des Zahnbehandlungsmittels bestimmt wird. Es ist hierbei denkbar, dass das Koordinatensystem direkt dem Zahnbehandlungsmittel zugeordnet ist, wobei zusätzlich oder alternativ auch denkbar ist, dass das Koordinatensystem zumindest teilweise und bevorzugt vollständig mittels definierten Körperoberflächenanteilen, insbesondere zwei oder mehreren Knöcheln der Hand, die den Übergang zwischen der Handfläche und den Fingern bilden, definiert wird.
Diese Ausführungsform ist vorteilhaft, da optisch zumindest über die Erfassung der Handbewegung zumindest indirekt zumindest teilweise eine Bewegung jedes beliebigen Zahnbehandlungsmittels, insbesondere jeder handelsüblichen Zahnbürste oder elektrischen Zahnbürste, erfassbar ist und die Verbreitung von optischen Erfassungsmitteln, insbesondere Handkameras, sehr hoch ist, wodurch die Erfindung für eine äußerst große Menge an Personen anwendbar ist. Besonders bevorzugt ist von der Datenverarbeitungseinrichtung das Zahnbehandlungsmittel von den übrigen Bildbestandteilen derart identifizierbar und zu separieren, dass dessen Bewegungen, insbesondere gegenüber oder relativ zu einem am Kopf oder im Bereich des Kopfes der Person festgelegten Bezugs- bzw. Koordinatensystems, erfassbar und besonders bevorzugt auswertbar sind. Weiterhin ist insbesondere zusätzlich bevorzugt mindestens oder genau ein Körperteil einer Person von der Datenverarbeitungseinrichtung von den übrigen Bildbestandteilen derart identifizierbar und separierbar, dass ein Bewegungsparameter des Zahnbehandlungsmittels gegenüber dem Körperteil bestimmbar bzw. berechenbar ist. Das Körperteil ist bevorzugt ein Teil eines Kopfes, wie z.B. Stirn, Augenbrauen, Augenhöhle, Mund, Lippen, Ohren, Wangen und/oder Kinn, bevorzugt ein oder mehrere körperfeste, insbesondere schädelfeste, Anteile wie z.B. die Stirn, das Kinn und/oder die Nase. Es ist jedoch ebenfalls denkbar, dass mehrere Körperteile, wie z.B. die Augen, der Mund und/oder die Nase erfasst werden. Es ist somit denkbar, dass ein oder mehrere Fotos von der Bedienperson bzw. dem Patienten, insbesondere vom Kopf bzw. dem Gesicht der Bedienperson, erfasst und gespeichert werden. Bevorzugt sind einzelne Körperformen, insbesondere Gesichtsbestandteilsformen bzw. Formen von charakteristischen Gesichtsmerkmalen manuell oder automatisch erfassbar oder festlegbar bzw. sind als Referenzpunkte oder Referenzflächen registrierbar, gegenüber denen ein oder mehrere Bewegungsparameter des Zahnbehandlungsmittels erfasst werden. Als Referenzpunkt können hierbei z.B. ein oder zwei Augen, die Nase, der Mund, ein oder zwei Ohren und/oder ein oder zwei Lippen herangezogen bzw. registriert werden. Weiterhin können zusätzlich oder alternativ die Augenbrauen oder die Augen, insbesondere die Pupillen, einer Person erfasst werden. Es kann dann eine die Augen bzw. Pupillen, insbesondere das Zentrum der Augen bzw. Pupillen, verbindende Linie oder Achse definiert bzw. festgelegt werden. Diese Linie oder Achse kann eine Richtung, insbesondere die X-, Y- oder Z-Richtung, eines Koordinatensystems definieren. Weiterhin kann eine zweite Linie bzw. Achse in der Erstreckungsrichtung der Nase, insbesondere des Nasenrückens, der Person definiert bzw. festgelegt werden, die bevorzugt eine zweite von der ersten Richtung abweichende Richtung, insbesondere die X-, Y- oder Z-Richtung, des Koordinatensystems definiert. Bevorzugt sind die erste Richtung und die zweite Richtung im Wesentlichen oder genau rechtwinklig zueinander ausgerichtet. Eine dritte Richtung erstreckt sich bevorzugt rechtwinklig gegenüber einer durch die erste und zweite Richtung aufgespannten Ebene. Die Erfassung der Bewegung des Zahnbehandlungsmittels gegenüber einem mit einer Bewegung des Kopfes gekoppeltem Koordinatensystem ist äußerst vorteilhaft, da die die Zahnbehandlung durchführende Person Kopf- und/oder Körperbewegungen durchführen kann, ohne dass die Erfassung der Bewegungsmuster ungenau oder unmöglich wird. Weiterhin bezieht sich die vorliegende Erfindung auf eine Verwendung eines Positions-, Geschwindigkeits- und/oder Beschleunigungssensors oder eines Videotrackingsystems zur Bestimmung eines sich während einer Zahnbehandlung ergebenden Bewegungsmusters eines Zahnbehandlungsmittels.
Die vorliegende Erfindung bezieht sich ferner auf ein Verfahren zum zumindest teilweisen Bestimmen eines sich bei einer Zahnbehandlung, insbesondere Zahnreinigung, ergebenden und auf die Zähne oder das Zahnfleisch einer die Zahnbehandlung erfahrenden Person mittels eines Zahnbehandlungsmittels ausgeübten Drucks. Das Verfahren umfasst bevorzugt mindestens die Schritte:
Durchführen einer zumindest mittelbaren und bevorzugt unmittelbaren optischen Erfassung von zumindest einem Teil des Zahnbehandlungsmittels bei der Zahnbehandlung mittels einer optischen Detektionseinrichtung bzw. Durchführen einer optischen Erfassung des Zahnbehandlungsmittels bei der Zahnbehandlung mittels einer optischen Detektionseinrichtung. Es ist hierbei bevorzugt denkbar, dass im Falle einer mittelbaren Erfassung des Zahnbehandlungsmittels eine Stellung und/oder Ausrichtung und/oder Haltung der Hand mittels der das Zahnbehandlungsmittel geführt wird, erfasst wird. Eine unmittelbare Erfassung des Zahnbehandlungsmittels beschreibt bevorzugt die tatsächliche optische Erfassung des Zahnbehandlungsmittels. Erzeugen von die optische Erfassung repräsentierenden Bilddaten, wobei die Bilddaten bevorzugt verschiedene Verformungsgrade des Zahnbehandlungsmittels aufweisen bzw. repräsentieren. Bevorzugt wird als Verformung eine Durchbiegung und/oder eine Torsion eines Zahnbürstenschafts und/oder eines Zahnbürstenkopfes erfasst. Zusätzlich oder alternativ ist jedoch ebenfalls denkbar, dass eine Verformung der Borsten einer Zahnbürste erfasst bzw. bestimmt wird. Auswerten der Bilddaten zur Bestimmung des jeweiligen Verformungsgrades des Zahnbehandlungsmittels. Ermitteln des Drucks, wobei der Druck zumindest in Abhängigkeit der Verformung des Zahnbehandlungsmittels und in Abhängigkeit weiterer Zahnbehandlungsmitteldaten bestimmt wird, wobei die Zahnbehandlungsmitteldaten Daten zu Eigenschaften des Zahnbehandlungsmittels umfassen.
Weiterhin ist denkbar, dass in Abhängigkeit des ermittelten Drucks über eine Ausgabeeinrichtung ein Signal oder eine Information ausgebbar ist. Bevorzugt repräsentiert das Signal oder die Information eine Handlungsempfehlung an den Benutzer des Zahnbehandlungsmittels, insbesondere an die Person, welche die Zahnbehandlung erfährt. Die Erfassung des Drucks bzw. die Ausgabe der in Abhängigkeit des detektierten Drucks auszugebenden Signale und/oder Informationen erfolgt bevorzugt in Echtzeit. Besonders bevorzugt erfolgt die Ausgabe der Signale und/oder Informationen über eine optische Ausgabeeinrichtung. Bevorzugt ist die optische Ausgabeeinrichtung Teil einer Vorrichtung zu der ebenfalls die optische Detektionseinrichtung gehört. Besonders bevorzugt werden die Signale und/oder Informationen zum Druck zeitgleich und/oder über dasselbe Ausgabegerät an dieselbe Person kommuniziert, mit dem ebenfalls Korrekturparametern zum Anpassen der Zahnbehandlungsbewegungen an eine Person, insbesondere die Person, die eine Zahnbehandlung erfährt und/oder durchführt, kommuniziert werden. Bei den Daten zu den Eigenschaften des Zahnbehandlungsmittels kann es sich um Materialeigenschaften und/oder Festigkeitswerte und/oder Abmessungen und/oder Biegesteifigkeiten und/oder Fabrikat und/oder Alter und/oder Benutzungshäufigkeit, insbesondere Benutzungsdauer, etc. handeln.
Gemäß einer weiteren bevorzugten Ausführungsform der vorliegenden Erfindung erfolgt die optische Erfassung eines Objekts, wobei zumindest die Erstreckung des Objekts in einer Dimension, d.h. in der Länge und/oder in der Breite und/oder in der Höhe und/oder im Umfang, bekannt ist, und die Erstreckung des Objekts in der bekannten Dimension bzw. in den bekannten Dimensionen wird zur Bestimmung von Dimensionsparametern verwendet, um geometrische Zusammenhänge, wie z.B. die Länge des bei einer Zahnbehandlung vom Zahnbehandlungsmittels zurückgelegten Wegs und/oder die Dimensionen der Zähne, insbesondere der sichtbaren Anteile der Zähne und/oder die Dimensionen des Zahnbehandlungsmittel und/oder den Abstand der Zähne und/oder des Zahnbehandlungsmittels zu der Detektionseinrichtung etc. zu erfassen. Das Objekt ist hierbei bevorzugt die Person, die das
Zahnbehandlungsmittel verwendet, wobei die bekannte Dimension der Person bevorzugt deren Größe ist. Zusätzlich oder alternativ ist denkbar, dass das Objekt das Zahnbehandlungsmittel, insbesondere die Zahnbürste, ist, wobei die bekannte Dimension der Zahnbürste bevorzugt die Länge und/oder die Breite und/oder die Höhe und/oder der Umfang ist.
Weiterhin kann sich die vorliegende Erfindung auf ein System oder Verfahren beziehen, das mindestens eine optische Erfassungseinrichtung, insbesondere mindestens eine Kamera, zum Erfassen von Daten zu mindestens einem Bewegungsparameter, insbesondere die Bewegungsrichtung, die Beschleunigung, der zurückgelegte Weg und/oder die Geschwindigkeit, eines Zahnbehandlungsmittels, und eine
Datenverarbeitungseinrichtung zur Separation eines das Zahnbehandlungsmittel repräsentierenden Bildbestandteils von mindestens einem weiteren durch die optische Erfassungseinrichtung erfassten Bildbestandteil und zum Bestimmen von mindestens einem zu dem Bewegungsparameter korrespondierenden Bewegungsmuster anhand der separierten Bildbestandteile, umfasst, wobei die optische Erfassungseinrichtung bevorzugt mindestens eine Kamera, insbesondere genau oder mindestens zwei Kameras, zur bevorzugt dreidimensionalen Erfassung des mindestens einen
Bewegungsparameters eines Zahnbehandlungsmittels aufweist, wobei die Prozessoreinrichtung bevorzugt ein dreidimensionales Koordinatensystem definiert, das sich bei einer Bewegung des Zahnbehandlungsmittels im Raum mit der das Zahnbehandlungsmittel führenden Hand mitbewegt, wobei die optische Erfassungseinrichtung bevorzugt einen Anteil des Kopfes einer Person erfasst und ein dreidimensionales Koordinatensystem in dem erfassten Anteil des Kopfs festlegt, wobei eine Bewegung des Zahnbehandlungsmittels bevorzugt in Abhängigkeit des festgelegten Koordinatensystems des Kopfes bestimmt wird oder wobei eine Bewegung des erfassten Anteils des Kopfes in Abhängigkeit des Koordinatensystems des Zahnbehandlungsmittels bestimmt wird, wobei die Prozessorvorrichtung bevorzugt ein Mobiltelefon oder ein TabletPC ist und die erfassten Daten bevorzugt über das Internet an eine Servereinrichtung zur weiteren Aufbereitung sendet und/oder über das Internet Daten zur Ausgabe mittels einer Ausgabeeinrichtung des Mobiltelefons oder des TabletPCs empfängt, wobei die Daten bevorzugt Informationen bezüglich Bewegungsparametern des Zahnbehandlungsmittels und/oder des Druck, mit dem das Zahnbehandlungsmittel an die Zähne und/oder das Zahnfleisch angepresst wird, umfassen.
Bevorzugt wird eine Datenbank bereitgestellt, die Zahnbehandlungsmitteldaten aufweist. Es ist hierbei denkbar, dass die Datenbank lokal auf einem Endgerät und/oder auf einem Server vorgehalten wird. Bevorzugt wird ein Identifikationsmittel, insbesondere ein softwarebasiertes Identifikationsmittel, bereitgestellt. Das Identifikationsmittel erfasst z.B. optisch das Zahnbehandlungsmittel oder das Zahnbehandlungsmittel repräsentierende Informationen, wie z.B. einen QR-Code. Das optisch erfasste Zahnbehandlungsmittel wird bevorzugt durch Bildinformationen bzw. Bilddaten repräsentiert, die bevorzugt mit in der Datenbank vorgehaltenen Bildinformationen bzw. Bilddaten zu einer Vielzahl an Zahnbehandlungsmitteln abgeglichen werden. In der Datenbank sind bevorzugt Daten bzw. Informationen zu den Eigenschaften der einzelnen Zahnbehandlungsmittel hinterlegt. Im Falle einer Übereinstimmung der in der Datenbank vorgehaltenen Daten bzw. Informationen mit den erfassten Bilddaten bzw. Bildinformationen erfolgt bevorzugt die Auswahl und Verwendung der in der Datenbank bzgl. dem konkreten bzw. identifizierten Zahnbehandlungsmittel hinterlegten Daten bzw. Informationen zur Ermittlung des Drucks. Eine Verknüpfung mit den einem Zahnbehandlungsmittel zuzurechnenden Daten bzw. Informationen, die in einer Datenbank vorgehalten werden, ist ferner über eine Verlinkung, wie sie z.B. durch die Auslesung eines Codes, insbesondere eines optischen Codes, wie z.B. eines Barcodes oder QR-Codes, möglich ist. Weiterhin ist zusätzlich oder alternativ denkbar, dass eine Maske zur manuellen Eingabe der Zahnbehandlungsmitteldaten bzw. - eigenschaften vorgesehen ist und die manuell über die Maske eingegebenen Daten bzw. Informationen zur Bestimmung des Drucks verwendet werden. Zusätzlich oder alternativ ist denkbar, dass mittels einer Funkeinrichtung, wie z.B. einer Nahfeldkommunikation bzw. Near-Field-Communication (NFC) oder mittels Bluetooth, die Daten bzw. Informationen bzgl. eines Zahnbehandlungsmittels in die Datenbank übertragen werden oder eine Verknüpfung zu der Datenbank erzeugt wird oder die Daten zur Ermittlung des Drucks hinterlegt werden. Ferner bezieht sich die Erfindung auf ein Computerprogrammprodukt zum Ausführen eines oder mehrere zuvor genannter Verfahren und/oder zur statistischen Auswertung der erzeugten Datensätze.
Die Begriffe optische Detektionseinrichtung und optische Erfassungseinrichtung können im Kontext dieser Erfindung als synonym verwendet verstanden werden.
Weitere Vorteile, Ziele und Eigenschaften vorliegender Erfindung werden anhand nachfolgender Beschreibung anliegender Zeichnungen erläutert, in welchen beispielhaft erfindungsgemäße Merkmale, Prozessoreinrichtungen oder Systeme zum Erfassen der Bewegungsmuster bei einer Zahnbehandlung dargestellt sind. Elemente der erfindungsgemäßen Einrichtungen und Verfahren, welche in den Figuren wenigsten im Wesentlichen hinsichtlich ihrer Funktion übereinstimmen, können hierbei mit gleichen Bezugszeichen gekennzeichnet sein, wobei diese Bauteile bzw. Elemente nicht in allen Figuren beziffert oder erläutert sein müssen. Nachfolgend wird die Erfindung rein beispielhaft anhand der beigefügten Figuren beschrieben. Darin zeigt:
Fig. 1a ein erfindungsgemäßes System zum Erfassen von Zahnbehandlungsbewegungen;
Fig. 1b ein erfindungsgemäßes internetbasiertes Netzwerk, in das mehrere erfindungsgemäße
   Systeme eingebunden sind;
Fig. 2a eine ausgestreckte Hand mit charakteristischen Oberflächenpunkten oder Oberflächenanteilen; und
Fig. 2b eine zur Faust geballte Hand mit charakteristischen Oberflächenpunkten oder Oberflächenanteilen, die ein Zahnbehandlungsmittel hält.

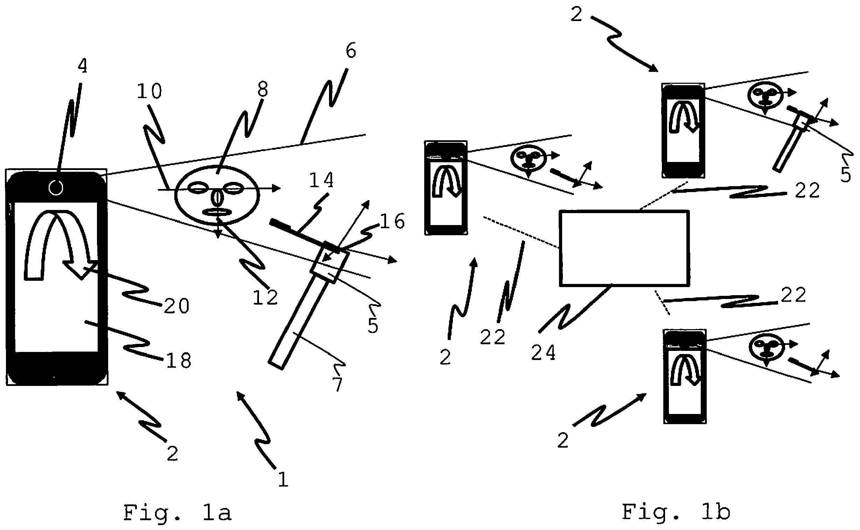

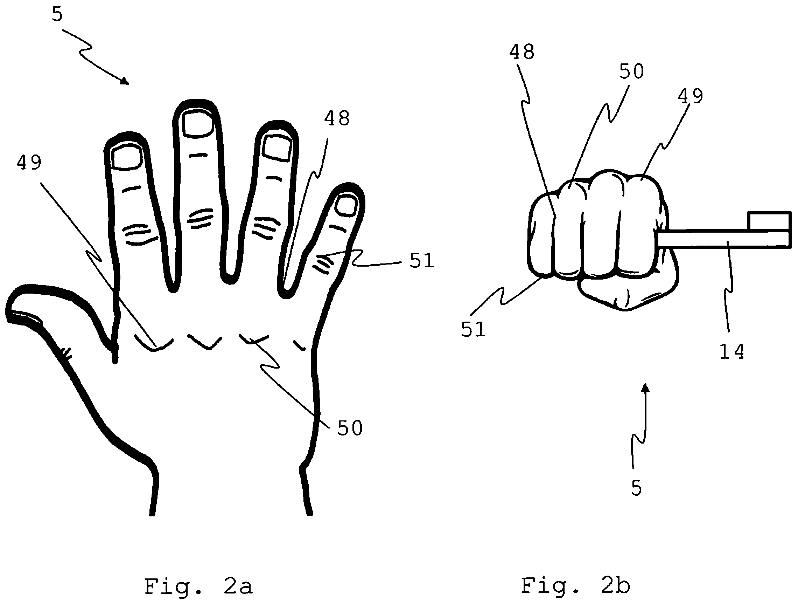

In Fig. 1a ist ein System 1 zum Erfassen von Zahnbehandlungsbewegungen mittels einer Prozessoreinrichtung 2 bzw. eines mobilen Endgeräts 2 gezeigt. Das mobile Endgerät 2, das bevorzugt als Mobiltelefon ausgebildet ist, weist dabei bevorzugt mindestens oder genau eine optische Detektionseinrichtung 4, insbesondere eine optische Erfassungseinrichtung 4, die bevorzugt als Kamera ausgebildet sein kann, auf. Die Kamera erfasst dabei bevorzugt genau oder mehr als 15 fps (frames per second bzw. Bilder pro Minute), genau, bis zu oder mehr als 30fps, genau, bis zu oder mehr als 45fps, genau, bis zu oder mehr als 60 fps, genau, bis zu oder mehr als 75 fps, genau, bis zu oder mehr als 90 fps, genau, bis zu oder mehr als 120fps oder genau, bis zu oder mehr als 200 fps. Das Bezugszeichen 6 kennzeichnet den Erfassungsbereich, in dem sich bevorzugt zumindest ein Teil des Kopfes, insbesondere eine oder beide Pupillen, die Nase und/oder der Mund, einer Person 8 sowie zumindest einen Teil des Zahnbehandlungsmittels 14 führenden Hand 5 befindet. Die Bezugszeichen 10 und 12 kennzeichnen bevorzugt personenfeste Achsen bzw. Koordinatenrichtungen, die bevorzugt genau einer Bewegung des Kopfes folgen. Das Bezugszeichen 14 kennzeichnet ein als Zahnbürste ausgebildetes
Zahnbehandlungsmittel, das gemäß Bezugszeichen 16 im Raum und somit gegenüber dem Kopf bewegt wird. Auf der optischen Ausgabeeinrichtung 18 der Prozessoreinrichtung 2 sind Bewegungsabläufe zur Korrektur der Bewegung und/oder zum Vorgeben eines Bewegungsablaufs anzeigbar.
In Fig. Ib ist gezeigt, dass mehrere Prozessoreinrichtungen 2 über eine Datenverbindung, insbesondere Internetverbindung, Daten an einen Server übermitteln, der bevorzugt aus den erfassten
Bewegungsmustern, insbesondere in Abhängigkeit von Krankheitshistorien oder Defekthistorien der einzelnen Personen, optimierte Bewegungscharakteristika erzeugt. Beiden Figuren Ia und Ib liegt das erfindungsgemäße Verfahren zum Bestimmen eines sich bei einer Zahnbehandlung, insbesondere Zahnreinigung, ergebenden Bewegungsmusters eines
Zahnbehandlungsmittels, insbesondere einer Zahnbürste, zugrunde. Das erfindungsgemäße Verfahren umfasst dabei mindestens die Schritte:
Bewegen des Zahnbehandlungsmittel zum Behandeln von Oberflächenanteilen der Zähne zumindest in einer X-/Y-Ebene, wobei mittels mindestens einer optischen Detektionseinrichtung 4 Daten bezüglich mindesten eines Bewegungsparameters, insbesondere die Bewegungsrichtung oder -rotation, die Beschleunigung, der Weg und/oder die Geschwindigkeit, des Zahnbehandlungsmittels 14 gegenüber einem sich mit dem Kopf 8 der behandelten Person mitbewegenden Bezugssystems 10, erfasst werden. Die Daten bestehen bevorzugt aus Bildinformationen, wie z.B. Pixelanordnungen, und bilden bevorzugt zumindest einen Teil des Kopfes 8 der Person und einen Teil der Hand 5 der Person, mit der die Person das Zahnbehandlungsmittel führt, ab. Das Bezugszeichen 7 kennzeichnet den Arm der Person, an der sich die das Zahnbehandlungsmittel 14 führende Hand 5 anschließt. Die Prozessoreinrichtung 2 definiert ein weiteres dreidimensionales Bezugssystem 16, das sich bei einer Bewegung des Zahnbehandlungsmittels 14 im Raum mit dem Zahnbehandlungsmittel 14 mitbewegt, wobei das weitere Bezugssystem 16 besonders bevorzugt durch charakteristische anthropometrische Körperpunkte der Hand, Körperlinien der Hand und/oder Körperoberflächen der Hand, mit der das Zahnbehandlungsmittel 14 geführt wird, gebildet wird. Dies ist vorteilhaft, da durch die Hand 5 des Nutzers das Zahnbehandlungsmittel 14 vollständig umschlossen werden kann und somit zumindest teilweise unsichtbar für die optische Detektionseinrichtung 4 sein kann. Basierend auf den Bewegungen der das weitere Bezugssystem 16 ausbildenden charakteristischen, insbesondere anthropometrischen, Körperpunkte der Hand, Körperlinien der Hand und/oder Körperoberflächen der Hand ist die Position und/oder Stellung des Zahnbehandlungsmittels 14 bestimmbar. Die Prozessoreinrichtung 2 wertet zur Definition des Weiteren dreidimensionalen Bezugssystems 16 bevorzugt die durch die optische Detektionseinrichtung 4 erfassten Daten, insbesondere Bildinformationen, aus.
Es ist jedoch ebenfalls denkbar, dass die von der optischen Detektionseinrichtung 4 erfassten Daten mittels einer Prozessoreinrichtung 2 aufbereitet bzw. ausgewertet werden, die außerhalb des mobilen Endgeräts ausgebildet ist. Bevorzugt ist hierbei denkbar, dass die Prozessoreinrichtung eine
Servereinrichtung ist, welche die Daten über eine Internetverbindung erhält. Bevorzugt sendet die als Servereinrichtung ausgebildete Prozessoreinrichtung die aufbereiteten oder modifizierten oder ausgewerteten Daten, oder darauf basierend erzeugte Daten, über eine Internetverbindung an das Endgerät.
Bereitstellen der erfassten Daten an die oder eine weitere Prozessoreinrichtung zur Bestimmung des Bewegungsmusters sowie bevorzugt die Bestimmung des Bewegungsmusters mittels der
Prozessoreinrichtung oder mittels der weiteren Prozessoreinrichtung. Es ist hierbei denkbar, dass z. B. mittels einer Prozessoreinrichtung eines mobilen oder stationären Endgeräts das weitere Bezugssystem definiert wird und die Bestimmung der Bewegungsmuster durch die Prozessoreinrichtung des mobilen Endgeräts erfolgt. Bevorzugt werden dabei die durch die optische Detektionseinrichtung 4 erfassten Daten hinsichtlich des sich mit dem Kopf 8 der Person mitbewegenden Bezugssystems 10 und dem sich mit der Hand 5 der Person mitbewegenden Bezugssystem 10 ausgewertet. Besonders bevorzugt werden die Relativbewegungen der Bezugssysteme 10, 16 zueinander bestimmt.
Bevorzugt wird zumindest zeitweise, insbesondere vor und/oder während des Zähneputzens, ferner die Stellung und/oder Position des Zahnbehandlungsmittels 14, insbesondere des Bürstenanteils der Zahnbürste, gegenüber der das Zahnbehandlungsmittel 14 führenden Hand 5 bestimmt. Bevorzugt erfolgt die Bestimmung der Position und/oder Stellung des Zahnbehandlungsmittels 14, insbesondere des Bürstenanteils der Zahnbürste, durch die Auswertung der durch die optische Detektionseinrichtung 4 bestimmten Daten. Fig. 2a zeigt den Handrücken einer Hand 5, wobei die Bezugszeichen 48, 49, 50 und 51 rein exemplarische charakteristische Punkt der Handoberfläche kennzeichnen. So kennzeichnet das Bezugszeichen 48 den Übergang zwischen zwei Fingern. Die Bezugszeichen 49 und 50 kennzeichnen die Knöchel der Hand und das Bezugszeichen 51 kennzeichnet einen Knöchel eines Fingers. Es wurde erkannt, dass diese Körperoberflächenpunkte bzw. -anteile und ähnliche Körperoberflächenpunkte bzw. -anteile jeweils sehr charakteristisch sind und sich daher für die optische Erfassung sehr gut eignen, wodurch die vorliegende Erfindung ein für den Nutzer sehr einfaches und funktional sehr belastbares Verfahren ermöglicht. In Fig. 2b ist die Hand 5 rein bespielhaft mit einem Zahnbehandlungsmittel 14 gezeigt. Dieser Darstellung lassen sich die einzelnen charakteristischen Körperpunkte bzw. - abschnitte entnehmen, wie sie bevorzugt von der optischen Detektionseinrichtung 4 erfasst werden.

Die vorliegende Erfindung kann sich somit auf ein Verfahren zum Bestimmen eines sich bei einer Zahnbehandlung, insbesondere Zahnreinigung, ergebenden Bewegungsmusters eines Zahnbehandlungsmittels, insbesondere einer Zahnbürste, beziehen, welches mindestens die Schritte umfasst:
- Bewegen des Zahnbehandlungsmittel zum Behandeln von Oberflächenanteilen der Zähne zumindest in einer X-/Y-Ebene, wobei mittels mindestens einer optischen Detektionseinrichtung Daten bezüglich mindesten eines Bewegungsparameters des Zahnbehandlungsmittels gegenüber einem sich mit dem Kopf der behandelten Person mitbewegenden Bezugssystems, erfasst werden, wobei die Daten charakteristische Körperpunkte der Hand, Körperlinien der Hand und/oder Körperoberflächen der Hand, mit der das Zahnbehandlungsmittel geführt wird, repräsentieren,
- Bereitstellen der erfassten Daten an eine Prozessoreinrichtung zur Bestimmung des Bewegungsmusters, wobei die Prozessoreinrichtung ein weiteres dreidimensionales Bezugssystem definiert, das sich bei einer Bewegung des Zahnbehandlungsmittels zumindest zeitweise im Raum mit dem Zahnbehandlungsmittel mitbewegt, wobei das weitere Bezugssystem durch die charakteristischen Körperpunkte der Hand, Körperlinien der Hand und/oder Körperoberflächen der Hand, mit der das Zahnbehandlungsmittel geführt wird, gebildet wird und
- Bestimmung des Bewegungsmusters, wobei zumindest eine Relativbewegung zwischen dem sich mit dem Kopf der behandelten Person mitbewegenden Bezugssystem und dem weiteren Bezugssystem bestimmt wird.
   Ferner kann sich die Erfindung auf ein Verfahren zur Bestimmung von mindestens einer Bewegungscharakteristik beziehen, welches mindestens die Schritte umfasst:
   Vielfaches Ausführen des zuvor genannten Verfahrens und Bestimmen mindestens einer übereinstimmenden Eigenschaft, insbesondere eines Bewegungs Parameters und/oder der Zahnbehandlungsdauer, der bestimmten Bewegungsmuster oder der erfassten Daten.

Ferner kann sich die Erfindung auf ein Verfahren zum Überwachen von Zahnbehandlungsbewegungen und zum Ausgeben von Korrekturparametern zum Anpassen der Zahnbehandlungsbewegungen beziehen, mindestens umfassend das Verfahren zum Bestimmen eines sich bei einer Zahnbehandlung, insbesondere Zahnreinigung, ergebenden Bewegungsmusters eines Zahnbehandlungsmittels, insbesondere einer Zahnbürste, welches mindestens die Schritte umfasst:
- Bewegen des Zahnbehandlungsmittel zum Behandeln von Oberflächenanteilen der Zähne zumindest in einer X-/Y-Ebene, wobei mittels mindestens einer optischen Detektionseinrichtung Daten bezüglich mindesten eines Bewegungsparameters des Zahnbehandlungsmittels gegenüber einem sich mit dem Kopf der behandelten Person mitbewegenden Bezugssystems, erfasst werden, wobei die Daten charakteristische Körperpunkte der Hand, Körperlinien der Hand und/oder Körperoberflächen der Hand, mit der das Zahnbehandlungsmittel geführt wird, repräsentieren,
- Bereitstellen der erfassten Daten an eine Prozessoreinrichtung zur Bestimmung des Bewegungsmusters, wobei die Prozessoreinrichtung ein weiteres dreidimensionales Bezugssystem definiert, das sich bei einer Bewegung des Zahnbehandlungsmittels zumindest zeitweise im Raum mit dem Zahnbehandlungsmittel mitbewegt, wobei das weitere Bezugssystem durch die charakteristischen Körperpunkte der Hand, Körperlinien der Hand und/oder Körperoberflächen der Hand, mit der das Zahnbehandlungsmittel geführt wird, gebildet wird und
- Bestimmung des Bewegungsmusters, wobei zumindest eine Relativbewegung zwischen dem sich mit dem Kopf der behandelten Person mitbewegenden Bezugssystem und dem weiteren Bezugssystem bestimmt wird
   und die Schritte:
   des Abgleichens der erfassten Bewegungsmuster mit in einer Datenbank vorgehaltenen Bewegungsmustern oder Bewegungscharakteristiken,
   Ableiten oder Bestimmen von Korrekturparametern, insbesondere mindestens einer Bewegungsrichtung, zur Anpassung der Bewegung des Zahnbehandlungsmittels in Abhängigkeit von den abgeglichenen Bewegungsmustern, und des Ausgebens einer Information mittels einer Ausgabeeinrichtung in Abhängigkeit von den abgeleiteten oder bestimmten
      Korrekturparametern.

Ferner kann bei einem zuvor genannten Verfahren, eine Bestimmung eines bei der Zahnbehandlung, insbesondere einer Zahnreinigung, mittels eines Zahnbehandlungsmittels, insbesondere einer Zahnbürste, auf die Zähne oder das Zahnfleisch der die Zahnreinigung erfahrenden Person ausgeübten Drucks erfolgten, wobei eine optische Erfassung des Zahnbehandlungsmittels mittels der optischen Detektionseinrichtung erfolgt, wobei durch die Detektionseinrichtung Bilddaten erzeugt werden und mittels der Bilddaten eine, insbesondere elastische, Verformung, insbesondere Durchbiegung, des Zahnbehandlungsmittels bestimmt wird, wobei der Druck zumindest in Abhängigkeit der Verformung des Zahnbehandlungsmittels bestimmt wird.

Ferner kann anhand der Relativbewegung zwischen dem sich mit dem Kopf der behandelten Person mitbewegenden Bezugssystem und dem weiteren Bezugssystem in Abhängigkeit von hinterlegten Zahnbürstenparametern ein beim Zähneputzen mittels der Zahnbürste auf die Zähne und/oder das Zahnfleisch ausgeübter Druck bestimmt werden.

Ferner kann eine Registrierung der zu behandelnden Zähne erfolgen, wobei die Registrierung die optische Erfassung mittels der Detektionseinrichtung oder eine manuelle Erfassung mittels einer Eingabemaske umfasst, wobei die Anzahl und/oder die Lage und/oder die Ausrichtung und/oder Defekte von zumindest einzelnen Zähnen erfasst werden, und wobei die Ableitung oder Bestimmung der Korrekturparameter bevorzugt unter Berücksichtigung der registrierten Zähne erfolgt.

Ferner kann mittels der optischen Detektionseinrichtung zur Bestimmung des sich mit dem Kopf der behandelten Person mitbewegenden Bezugssystem mindestens drei bevorzugt vorgegebene Punkte eines Kopfes, einer Person erfasst werden, wobei mindestens zwei Punkte mit einer Linie verbindbar sind und mindesten ein dritter Punkt nicht auf dieser Linie liegt, wobei durch die mindesten drei erfassten Punkte das Bezugssystem definiert wird und wobei eine Bewegung des Zahnbehandlungsmittels gegenüber diesem Bezugssystem erfasst wird und/oder
wobei mittels der optischen Detektionseinrichtung mindestens zur Bestimmung des weiteren Bezugssystems drei bevorzugt vorgegebene Punkte der das Zahnbehandlungsmittel, insbesondere eine Zahnbürste, führenden Hand erfasst werden, wobei mindestens zwei Punkte mit einer Linie verbindbar sind und mindesten ein dritter Punkt nicht auf dieser Linie liegt, wobei durch die mindesten drei erfassten Punkte das Bezugssystem definiert wird und wobei eine Bewegung des Kopfes gegenüber diesem weiteren Bezugssystem erfasst wird.

Ferner kann in Abhängigkeit einer Bewegung des Körpers der Person gegenüber der optischen
Detektionseinrichtung voneinander unterschiedliche körperfeste Oberflächenpunkte, insbesondere des Kopfes, der Person zur Definition des Bezugssystems erfasst werden. Ferner kann die optische Erfassung eines Objekts erfolgen, wobei zumindest die Erstreckung des Objekts in einer Dimension, d.h. in der Länge und/oder in der Breite und/oder in der Höhe, bekannt ist, und die Erstreckung des Objekts in der bekannten Dimension bzw. in den bekannten Dimensionen zur Bestimmung von Dimensionsparametern verwendet wird, um geometrische

Zusammenhänge, wie z.B. die Länge des bei einer Zahnbehandlung vom Zahnbehandlungsmittels zurückgelegten Wegs, zu erfassen.

Ferner kann sich die vorliegende Erfindung auf ein Computerprogrammprodukt und System zum Ausführen eines zuvor genannten Verfahrens beziehen.

Ferner kann sich die Erfindung auf ein Verfahren zum zumindest teilweisen Bestimmen eines sich bei einer Zahnreinigung ergebenden Bewegungsmusters eines Zahnbehandlungsmittels, insbesondere einer Zahnbürste, beziehen, wobei das Verfahren zumindest den Schritt des optischen Erfassens von Daten mittels mindestens einer Kamera zu mindestens einem Bewegungsparameter eines Zahnbehandlungsmittels und den Schritt der bildlichen Separation des Zahnbehandlungsmittels, insbesondere der das Zahnbehandlungsmittel führenden Hand, von mindestens einem weiteren durch die Kamera erfassten Bildbestandteil mittels einer Datenverarbeitungseinrichtung und den Schritt des Bestimmens von mindestens einem zu dem Bewegungsparameter korrespondierenden Bewegungsmuster anhand der separierten Bildbestandteile aufweist, wobei das Zahnbehandlungsmittel zusätzlich mit einer oder mehreren Sensoreinrichtungen, die einen Positions-, Geschwindigkeits-, Beschleunigungs- und/oder Rotationssensor umfassen, ausgestattet ist, wobei die Datenverarbeitungseinrichtung zur Bestimmung des Bewegungsparameters die Sensordaten und die Daten der Kamera verwendet, wobei das Zahnbehandlungsmittel eine elektrische Zahnbürste ist und die eine Sensoreinrichtung oder die mehreren Sensoreinrichtungen fest mit der elektrischen Zahnbürste verbunden ist/sind.

Ferner kann das Zahnbehandlungsmittel und die das Zahnbehandlungsmittel führende Hand von mindestens einem weiteren durch die optische Erfassungseinrichtung erfassten Bildbestandteil mittels einer Datenverarbeitungseinrichtung separiert werden.

Ferner kann mindestens oder genau ein Körperteil oder mehrere Körperteile einer Person von der Datenverarbeitungseinrichtung von den übrigen Bildbestandteilen derart identifizierbar und separierbar sein, dass ein Bewegungsparameter des Zahnbehandlungsmittels gegenüber dem Körperteil bestimmbar ist, wobei das Körperteil ein Teil eines Kopfes, nämlich die Stirn, Augenbrauen, Augenhöhle, Lippen, Ohren, Wangen und/oder Kinn und/oder die Nase ist, wobei die mehreren Körperteile die Augen, der Mund und/oder die Nase sind.

Ferner kann das Zahnbehandlungsmittel mindestens eine Leitstützstruktur umfassen, wobei an der Leitstützstruktur eine Behandlungseinrichtung zum zumindest mittelbaren Inkontaktbringen mit einer Zahnoberfläche und ein Kontaktbereich zum Halten des Zahnbehandlungsmittels angeordnet oder ausgebildet sind, wobei die eine Sensoreinrichtung ein Beschleunigungs- oder Rotationssensor ist oder die mehreren Sensoreinrichtungen Beschleunigungs- und Rotationssensoren sind.

Ferner kann sich in einem Erfassungsbereich der Kamera zumindest eine oder beide Pupillen, die Nase und der Mund der behandelten Person sowie zumindest ein Teil der das Zahnbehandlungsmittels führenden Hand befinden.

Ferner kann in oder an dem Zahnbehandlungsmittel ein Drucksensor zur Erfassung des mittels des Zahnbehandlungsmittels auf die Zähne und/oder das Zahnfleisch ausgeübten Anpressdrucks vorgesehen sein.

Ferner kann das Zahnbehandlungsmittel eine Kommunikationsschnittstelle zum Übertragen der erfassten Daten, insbesondere in roher oder aufbereiteter Form, an die Datenverarbeitungseinrichtung aufweisen, wobei die Kommunikationsschnittstelle derart gestaltet ist, dass die Daten kabellos übertragen werden.

Ferner kann sich die Erfindung auf ein System zum Ausführen eines zuvor genannten Verfahrens beziehen, wobei das System umfasst: Eine elektrische Zahnbürste; eine Sensoreinrichtung oder mehrere Sensoreinrichtungen, die einen Positions-, Geschwindigkeits-, Beschleunigungs- und/oder Rotationssensor umfassen, die fest mit der elektrischen Zahnbürste verbunden ist/sind; mindestens eine Kamera zum optischen Erfassen von Daten zu mindestens einem Bewegungsparameter der elektrischen Zahnbürste; eine Prozessorvorrichtung und eine Datenverarbeitungseinrichtung zur bildlichen Separation der elektrischen Zahnbürste von mindestens einem weiteren durch die mindestens eine Kamera erfassten Bildbestandteil und zum Bestimmen von mindestens einem zu dem Bewegungsparameter korrespondierenden Bewegungsmuster, wobei die Sensordaten und die Daten der Kamera durch die Datenverarbeitungseinrichtung zur Bestimmung des Bewegungsparameters verwendet werden.

Ferner kann sich die Erfindung auf ein Computerprogrammprodukt beziehen, welches Befehle umfasst, die bewirken, dass ein
System umfassend eine elektrische Zahnbürste; eine Sensoreinrichtung oder mehrere Sensoreinrichtungen, die einen Positions-, Geschwindigkeits-, Beschleunigungs- und/oder Rotationssensor umfassen, die fest mit der elektrischen Zahnbürste verbunden ist/sind; mindestens eine Kamera zum optischen Erfassen von Daten zu mindestens einem Bewegungsparameter der elektrischen Zahnbürste; eine Prozessorvorrichtung und eine Datenverarbeitungseinrichtung zur bildlichen Separation der elektrischen Zahnbürste von mindestens einem weiteren durch die mindestens eine Kamera erfassten Bildbestandteil und zum Bestimmen von mindestens einem zu dem Bewegungsparameter korrespondierenden Bewegungsmuster, wobei die Sensordaten und die Daten der Kamera durch die Datenverarbeitungseinrichtung zur Bestimmung des Bewegungsparameters verwendet werden,
die Verfahrensschritte des
Verfahrens zum zumindest teilweisen Bestimmen eines sich bei einer Zahnreinigung ergebenden Bewegungsmusters eines Zahnbehandlungsmittels, insbesondere einer Zahnbürste,
auszuführen,
wobei das Verfahren zumindest den Schritt des optischen Erfassens von Daten mittels mindestens einer Kamera zu mindestens einem Bewegungsparameter eines Zahnbehandlungsmittels und den Schritt der bildlichen Separation des Zahnbehandlungsmittels, insbesondere der das Zahnbehandlungsmittel führenden Hand, von mindestens einem weiteren durch die Kamera erfassten Bildbestandteil mittels einer Datenverarbeitungseinrichtung und den Schritt des Bestimmens von mindestens einem zu dem Bewegungsparameter korrespondierenden Bewegungsmuster anhand der separierten Bildbestandteile aufweist, wobei das Zahnbehandlungsmittel zusätzlich mit einer oder mehreren Sensoreinrichtungen, die einen Positions-, Geschwindigkeits-, Beschleunigungs- und/oder Rotationssensor umfassen, ausgestattet ist, wobei die Datenverarbeitungseinrichtung zur Bestimmung des Bewegungsparameters die Sensordaten und die Daten der Kamera verwendet, wobei das Zahnbehandlungsmittel eine elektrische Zahnbürste ist und die eine Sensoreinrichtung oder die mehreren Sensoreinrichtungen fest mit der elektrischen Zahnbürste verbunden ist/sind.

## Patentansprüche

1. System zum Erfassen von Zahnbehandlungsbewegungen,
aufweisend eine Zahnbürste, wobei die Zahnbürste mindestens eine Leitstützstruktur umfasst, wobei an der Leitstützstruktur eine Behandlungseinrichtung zum zumindest mittelbaren Inkontaktbringen mit einer Zahnoberfläche und ein Kontaktbereich zum Halten des Zahnbehandlungsmittels angeordnet oder ausgebildet sind, wobei ein Beschleunigungs- und/oder Rotationssensor zur Erfassung von Bewegungsparametern zumindest während einer Verwendung der Zahnbürste mit der Leitstützstruktur körperlich verbunden ist, wobei die Zahnbürste eine elektrische Zahnbürste ist und der Beschleunigungs- und/oder Rotationssensor fest mit der elektrischen Zahnbürste verbunden ist, wobei die elektrische Zahnbürste eine Kommunikationsschnittstelle zum Übertragen der erfassten Daten an eine Datenverarbeitungseinrichtung aufweist, wobei die Kommunikationsschnittstelle derart gestaltet ist, dass die Daten kabellos übertragbar sind,
wobei in oder an der elektrischen Zahnbürste ein Drucksensor zur Erfassung des mittels der elektrischen Zahnbürste auf den Mund bzw. die Zähne und/oder das Zahnfleisch ausgeübten Anpressdrucks vorgesehen ist,
wobei in Abhängigkeit des ermittelten Drucks über eine Ausgabeeinrichtung ein Signal oder eine Information ausgebbar ist, wobei die Ausgabe der in Abhängigkeit des detektierten Drucks auszugebenden Signale und/oder Informationen in Echtzeit erfolgt, wobei die Ausgabe der Signale und/oder Informationen über eine optische Ausgabeeinrichtung erfolgt,
wobei die Datenverarbeitungseinrichtung eine mit dem Internet zumindest zeitweise verbindbare Einrichtung nämlich ein Tabletcomputer oder ein Mobiltelefon ist.

2. System nach Anspruch 1,
**dadurch gekennzeichnet, dass**
das Mobiltelefon oder der TabletPC über das Internet Daten zur Ausgabe mittels einer Ausgabeeinrichtung des Mobiltelefons oder des TabletPCs empfängt, wobei die Daten bevorzugt Informationen bezüglich Bewegungsparametern der elektrischen Zahnbürste und/oder des Drucks, mit dem die elektrische Zahnbürste an die Zähne und/oder das Zahnfleisch angepresst wird, umfassen.

3. System nach Anspruch 2,
**dadurch gekennzeichnet, dass**
eine Personalisierungsinstanz vorgesehen ist, wobei
die erfassten Bewegungsparameter einer konkreten Person mittels der Personalisierungsinstanz nämlich einer Telefonnummer oder einer Emailadresse dieser konkreten Person zuordenbar sind.

4. System nach Anspruch 3,
**dadurch gekennzeichnet, dass**
eine Datenbank bereitgestellt ist, wobei die Datenbank Zahnbehandlungsmitteldaten aufweist, wobei die Datenbank lokal auf dem Mobiltelefon oder TabletPC und/oder auf einem Server vorgehalten wird.

5. System nach Anspruch 4,
**dadurch gekennzeichnet, dass**
die Zahnbehandlungsmitteldaten Daten zu dem Fabrikat umfassen.

6. System nach Anspruch 4 oder 5,
**dadurch gekennzeichnet, dass**
die Zahnbehandlungsmitteldaten Daten zu dem Alter umfassen.

7. System nach Anspruch 4 oder 5 oder 6,
**dadurch gekennzeichnet, dass**
die Zahnbehandlungsmitteldaten Daten zu der Benutzungshäufigkeit, insbesondere Benutzungsdauer, umfassen.

8. System nach einem der Ansprüche 3 bis 7,
**dadurch gekennzeichnet, dass**
das Mobiltelefon oder der TabletPC mindestens oder genau eine Kamera aufweist.

9. System nach Anspruch 8,
**dadurch gekennzeichnet, dass**
die optische Ausgabeeinrichtung Teil des Mobiltelefons oder TabletPCs ist, zu dem ebenfalls die Kamera gehört, wobei Signale und/oder Informationen zum Druck zeitgleich und/oder über dasselbe Ausgabegerät an dieselbe Person kommunizierbar sind, mit dem ebenfalls Korrekturparametern zum Anpassen der Zahnbehandlungsbewegungen an eine Person, insbesondere die Person, die eine Zahnbehandlung erfährt und/oder durchführt, kommunizierbar sind.

10. System nach Anspruch 8 oder 9,
**dadurch gekennzeichnet, dass**
ein oder mehrere Fotos von der Person, insbesondere vom Kopf bzw. dem Gesicht der Person, erfassbar und speicherbar sind.

11. System nach Anspruch 8 oder 9 oder 10,
**dadurch gekennzeichnet, dass**
in einem Erfassungsbereich der Kamera zumindest ein Teil des Kopfes nämlich eine oder beide Pupillen, die Nase und der Mund der Person 8 sowie zumindest ein Teil der die elektrische Zahnbürste führenden Hand 5 anordenbar sind.

12. System nach Anspruch 11,
**dadurch gekennzeichnet, dass**
von dem Mobiltelefon oder dem TabletPC zur Bestimmung des Bewegungsparameters die Sensordaten und die Daten der Kamera verwendbar sind.
